# EUROPEAN PATENT SPECIFICATION

(11) **EP 1 628 995 B1**
(45) Date of publication and mention of the grant of the patent: **27.06.2007**
(21) Application number: 04731809.2
(22) Date of filing: 07.05.2004
(51) Int. Cl.: C07K 1/10, C07K 5/02, C07K 5/06, C07D 209/42, C07D 249/18, C07D 471/04, C07C 275/70

(54) **A PROCESS FOR THE PREPARATION OF PERINDOPRIL USING TETRAMETHYLURONIUM SALTS AS COUPLING REAGENTS**
VERFAHREN ZUR HERSTELLUNG VON PERINDOPRIL UNTER VERWENDUNG VON TETRAMETHYLURONIUMSALZEN ALS KOPPLUNGSREAGENZIEN
PROCEDE DE PREPARATION DE PERINDOPRIL UTILISANT DES SELS DE TETRAMETHYLURONIUM COMME REACTIFS DE COUPLAGE

(30) Priority: 08.05.2003 SI 200300118
(43) Date of publication of application: 01.03.2006
(73) Proprietor: LEK Pharmaceuticals d.d., 1526 Ljubljana (SI)
(72) Inventor: RUCMAN, Rudolf, 1211 Ljubljana-Smartno (SI)
(74) Representative: Dietz, Jörg-Reimar
(86) International application number: PCT/SI2004/000020
(87) International publication number: WO 2004/099236

(56) References cited:
- EP-A- 1 279 665
- WO-A-02/094857

## Description

### Field of the invention

The present invention belongs to the field of the organic chemistry synthesis and relates to a process for the preparation of the ACE inhibitor perindopril.
In particular, the present invention relates to the simple and highly selective process for the preparation of perindopril, useful also for the industrial production using commercially available raw materials and reagents.

### Prior art

Perindopril was first produced by a process described in EP-A-0049658, wherein a four stage process which leads to the formation of stereoisomers that have to be separated using complex methods is disclosed. The reagent for condensation of both crucial intermediates was dicyclohexylcarbodiimide. Patent EP-0308339 B1 relates to the preparation of crucial starting raw material for the synthesis of perindopril, that is (2S,3aS,7aS)-octahydroindole-2-carboxylic acid.

US 4,914,214 describes the process for the preparation of the same starting raw material by hydrogenation of (S)-2-carboxyindoline. The compound obtained by hydrogenation is then condensed with N-/1-(S)-ethoxycarbonyl-butyl/-(S)-alanine using dicyclohexylcarbodiimide as a reagent for condensation and 1-hydroxybenzotriazole as an auxiliary nucleophilic reagent. The drawback of this process is the formation of dicyclohexylurea which is difficult to remove from the reaction mixture.

An alternative process for the preparation of perindopril is described in patent EP-1279665 A2. In the first stage of the process N-/1-(S)-ethoxycarbonyl-butyl/-(S)-alanine is activated with phosgene or polymeric phosgene forming the corresponding oxazolidine which further reacts with (2S,3aS,7aS)-octahydroindolo-2-carboxylic acid. A further aspect of the invention is the condensation using N,N'-carbonyldiimidazole. It is of interest that in this process the carboxy side group is unprotected. It is, otherwise, well known from the peptide chemistry that the condensation of amino acids with unprotected side groups increases the potential for the side reactions.

The aim of this invention is to prepare perindopril and pharmaceutically acceptable salts thereof, such as t-butylamine salt, in a new and simple manner, wherein perindopril is obtained in a high yield and of high purity.

### Detailed description of the invention

The first embodiment of the present invention is the process for the preparation of perindopril of the formula I: 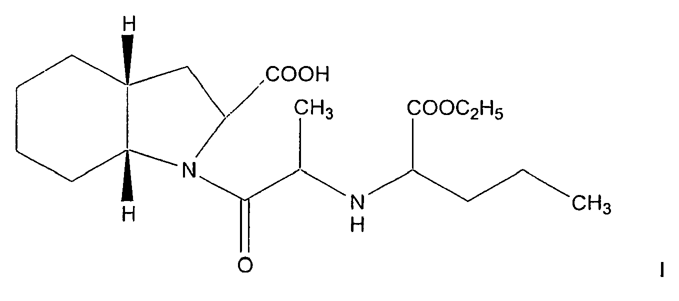 characterized in that the carboxy group of stereospecific amino acid N-/1-(S)-ethoxycarbonyl-butyl/-(S)-alanine of the formula II: 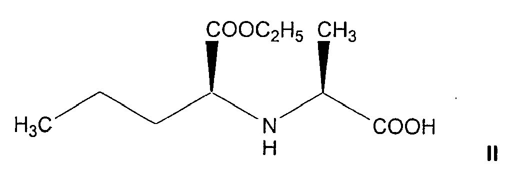 is activated with tetramethyluronium salt of the formula III: 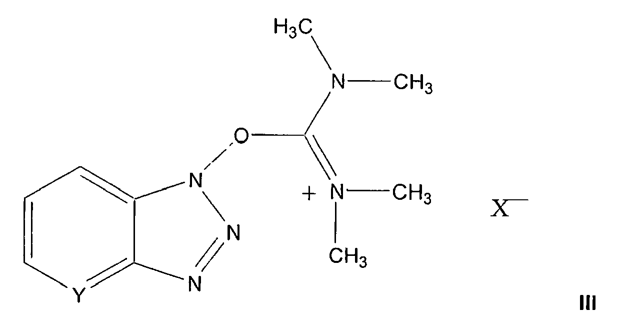 wherein Y is an aromatic C or N atom, X⁻ is an anion as tetrafluoroborate, hexafluorophosphate or halogen,
and then the obtained activated amino acid of the formula II is reacted with (2S,3aS,7aS)-octahydroindolo-2-carboxylic acid or ester thereof, of the formula IV: 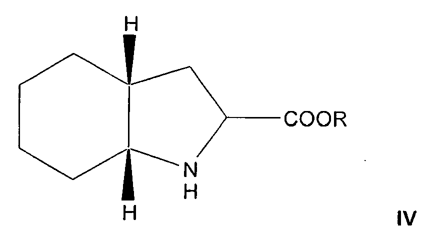 wherein R is a hydrogen, benzylic group, tertiary butylic group or trimethylsilyl group.

A tetramethyluronium salt may be selected from the group consisting of:
O-(benzotriazol-1-yl)-N,N,N',N'-tetramethyluronium hexafluorophosphate, abbreviated: HBTU;
O-(benzotriazol-1-yl)-N,N,N',N'-tetramethyluronium tetrafluoroborate, abbreviated: TBTU; and
O-(7-aza-benzotriazol-1-yl)-N,N,N',N'-tetramethyluronium hexafluorophosphate, abbreviated: HATU,
which are commercially available.
These reagents are known from the literature (G.A.Grant, Synthetic Peptides, Oxford University Press, 1992, 119) as suitable reagents for the synthesis of peptides without side reactions of isomerisation on chiral centres.

In the second embodiment of the present invention the process for the preparation of the perindopril is characterised in that in the step of activation of the amino acid of the formula II tertiary organic base is added. Two moles of tertiary organic base are added to 1 mole of equimolar mixture of tetramethyluronium reagent and an acidic compound of the formula II.
The reaction of activation is disclosed in the literature (Aldrichimica Acta, Vol.29,No.2, 1996, p.9) and comprises the formation of highly reactive intermediate of the formula V: 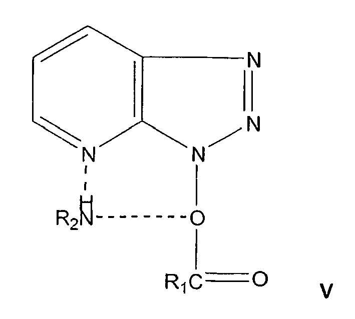 which reacts with an amine compound, e.g. with (2S, 3aS,7aS)-octahydroindolo-2-carboxylic acid or an ester thereof, and is converted into a dipeptide, e.g. perindopril.

Tertiary organic base according to the present invention may be a tertiary amine selected form the group consisting of triethylamine, pyridine, lutidine and N,N-diisopropylethylamine. Preferably, a tertiary amine is N,N-diisopropylethylamine.

In third embodiment of the present invention the process for the preparation of the perindopril is characterised in that in the step of activation of the amino acid of the formula II the basic reaction solvent can be used, e.g. 1-methylimidazole.
Otherwise, a solvent for the process for the preparation of perindopril according to present invention may be selected form the group consisting of N,N-dimethylformamide, N,N-dimethylacetamide, N-methyl-pyrrolidone, dichloromethane or mixture thereof.

Perindopril may be isolated from the final reaction mixture by the extraction with dichloromethane or ethylacetate. All side products and reagents are water soluble and may be removed from the reaction mixture by simple extraction with water solutions. Finally, the resulting organic phase containing perindopril is dried by evaporation of the solvent in vacuum.

When in the process of the present invention esters of (2S,3aS,7aS)-octahydroindolo-2-carboxylic acid are used for the reaction with activated amino acid, the protective ester group must be removed at the end of the process. The benzylic protective group may be removed by hydrogenation using hydrogen/catalyst phase transfer (CTH) method with palladium catalyst on charcoal and addition of a proton donor, e.g. ammonium formate. In this case, use of gaseous hydrogen can be avoided thereby decreasing the possibility of ignition or explosion in an industrial process. Tertiary butyl protective group may be removed in the phase of isolation by extraction with hydrochloric acid. Trimethylsilyl protective group may be removed in the phase of extraction in contact with water.

Perindopril is obtained as colourless viscous oil which solidifies in cold. Perindopril may be converted into perindopril erbumin by crystallisation after addition of t-butylamine from ethylacetate or acetonitrile according to the known method.

The invention is illustrated but not in any way limited by the following examples:

### Example 1

### Preparation of benzyl-perindopril

N-/1-(S)-ethoxycarbonyl-butyl/-(S)-alanine (7.2 g, 33 mmoles) and TBTU (11.8 g, 36.7 mmoles) were suspended in a solvent mixture of 40 ml of N,N-dimethylformamide and 10 ml of dichloromethane. N,N-di-isopropylethylamine (12.5 ml, 73 mmoles) was added with stirring and the mixture was continually stirred for 10 min until complete dissolution. With continuous vigorous stirring at room temperature, 30 ml of a solution of (3S, 3aS,7aS)-octahydroindolo-2-carboxylic acid benzyl ester (8.63 g, 33 mmoles) in dichloromethane was added to the reaction mixture. Stirring in nitrogen atmosphere was continued for further 4 hours.

The reaction can be monitored by HPLC chromatography using the following parameters:
column: Kromasil 100, C18, 5µ, 150 x 4.6 mm
mobile phase:
   - A -: 20% acetonitrile/80% buffer with 0.1% of triethylamine/phosphoric acid, pH 3.2
   - B -: 60% acetonitrile/40% buffer with 0.1% of triethylamine/phosphoric acid, pH 3.2.
gradient: from 100% A to 50% A in 20 min.
flow rate: 1 ml/min
detection: UV, 215 nm
Retention times: benzyl-perindopril: 5.7 min; perindopril: 11.7 min.
After completion of the reaction, 300 ml of dichloromethane was added to the final reaction mixture. The obtained mixture was extracted with two 400-ml portions of 5% saline, 300 ml of 2% hydrochloric acid in water, 300 ml of water, two 300-ml portions of saturated sodium hydrogencarbonate and finally with 300 ml of water. The dichloromethane phase was then treated with magnesium sulphate, filtered and dried by evaporation of the solvent at max. 50 °C in vacuum to obtain benzyl-perindopril as slightly yellowish oil (13.5 g, 98% of the theory).

### Example 2

### Preparation of perindopril

### a.) Hydrogenation with phase transferred hydrogen (CTH)

Benzyl-perindopril from example 1 (13.5 g) was dissolved in 300 ml of methanol, to the solution were added 1.35 g of catalyst (10% palladium on charcoal) and 1.35 g of ammonium formate. The mixture was stirred at room temperature for 30 min. Then the catalyst was filtered off and washed with 50 ml of methanol. The resulting solution was evaporated in vacuum at 50 °C. The dry residue was dissolved in 100 ml of dichloromethane and extracted with: two 100-ml portions of 5% saline, two 100-ml portions of water. The dichloromethane phase was then treated with magnesium sulphate, filtered and dried by evaporation of dichloromethane at 50 °C in vacuum. The residue consisted of a crude, clear and colourless oil which solidified in cold - perindopril (9.8 g, 90%). The obtained substance was identical with the standard compound - perindopril (HPLC, IR and MS spectra).

### b.) Classical hydrogenation with hydrogen gas

Benzyl-perindopril from example 1 (13.5 g) was dissolved in 300 ml of methanol, to the solution was added 1.35 g of catalyst (10% palladium on charcoal). The mixture was stirred at room temperature under moderate flow of hydrogen for further 5 hours. The catalyst was then filtered off, washed with 50 ml of methanol and the solution was evaporated at 50 °C in vacuum. The residue was a clear, colourless oily compound which solidified in cold and was identical with the sample of perindopril (10.2 g, 94% of the theory).

### Example 3

### Preparation of perindopril

N-/1-(S)-ethoxycarbonyl-butyl/-(S)-alanine (7.2 g, 33 mmoles) and HATU (13.95 g, 36.7 mmoles) were suspended in 25 ml of dry N-methylpyrrolidone and 25ml of dry dichloromethane. To the mixture was added triethylamine (10.2 ml, 73 mmoles, previously dried with NaOH and distilled in the presence of ninhydrin) with continuous stirring. After 10 min of stirring, the solution of (2S,3aS,7aS)-octahydroindolo-2-carboxylic acid trimethylsilyl ester (8.0 g, 33 mmoles) in 30 ml of dry dichloromethane was added. The solution was stirred for further 4 hours at room temperature. The reaction mixture was then diluted with 250 ml of dichloromethane and extracted with: two 400-ml portions of 5% saline, two 300-ml portions of 2% hydrochloric acid in water, 300 ml of water, 300 ml of saturated ice-cold sodium hydrogencarbonate and finally 300 ml of ice-cold water. The obtained dichloromethane phase was treated with magnesium sulphate, filtered and dried by evaporation of the solvent at 50 °C in vacuum. 9 g (83% of the theory) of a clear, colourless oil which solidified in cold was obtained, identical with perindopril (HPLC, IR, MS).

## Claims

1. A process for the preparation of perindopril of the formula I: 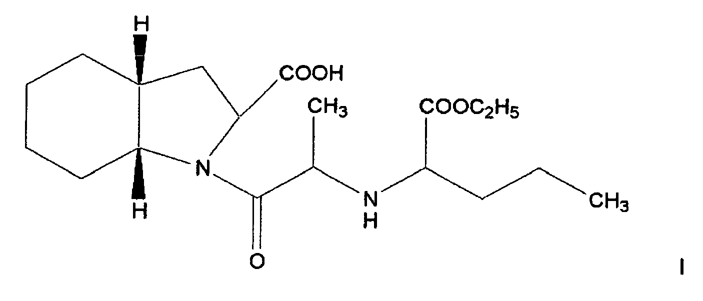 **characterised in that** the carboxy group of stereospecific amino acid N-/1-(S)-ethoxycarbonyl-butyl/-(S)-alanine of the formula II: 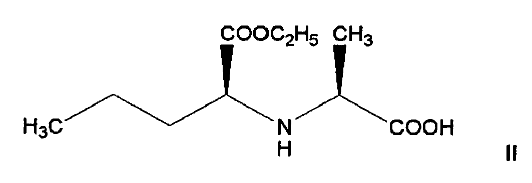 is activated with tetramethyluronium salt of the formula III: 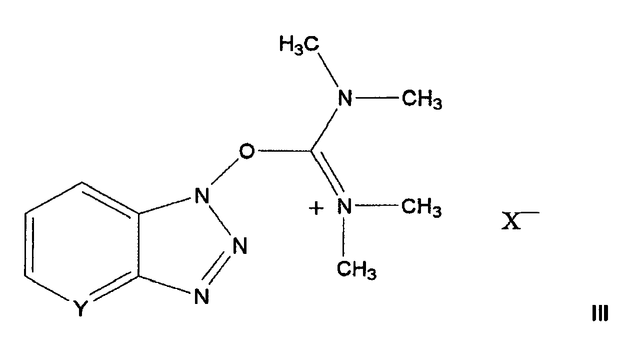 wherein Y is an aromatic C or N atom, X⁻ is an anion as tetrafluoroborate, hexafluorophosphate or halogen,
and then the obtained activated amino acid of the formula II is reacted with (2S,3aS,7aS)-octahydroindole-2-carboxylic acid or ester thereof, of the formula IV: 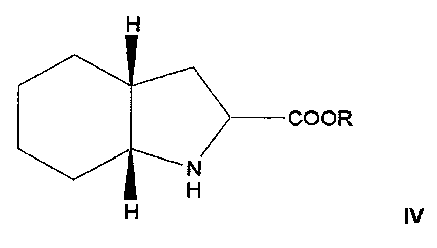 wherein R is hydrogen, benzylic group, tertiary butylic group or trimethylsilyl group,
and after completion of the reaction the protecting group R is removed by hydrogenation, phase transfer hydrogenation or extraction.

2. The process according to claim 1, **characterised in that** the tetramethyluronium salt is selected from the group consisting of
O-(benzotriazol-1-yl)-N,N,N',N'-tetramethyluronium hexafluorophosphate,
O-(benzotriazo)-1-yl)-N,N,N',N'-tetramethyluronium tetrafluoroborate, and
O-(7-aza-benzotriazol-1-yl)-N,N,N',N'-tetramethyluronium hexafluorophosphate.

3. The process according to any one of claims 1 to 2, **characterised in that** the activation of carboxylic group with tetramethyluronium salts is promoted by the use of tertiary organic base.

4. The process according to claim 3, **characterised in that** the tertiary organic base is selected from the group consisting of N,N-diisopropyl-ethylamine and triethylamine.

5. The process according to claim 1, **characterised in that** the protecting group R is benzylic group.

6. The process according to claim 5, **characterised in that** the benzylic protecting group is removed by phase transfer hydrogenation without use of gaseous hydrogen.

## Patentansprüche

1. Verfahren zur Herstellung von Perindopril der Formel I 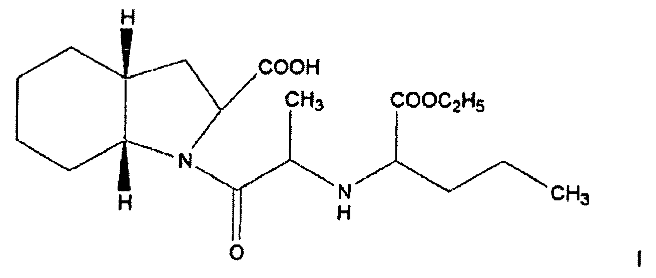 **dadurch gekennzeichnet, dass** die Carboxygruppe der stereospezifischen Aminosäure N-/1-(S)-Ethoxycarbonylbutyl/-(S)-alanin der Formel II 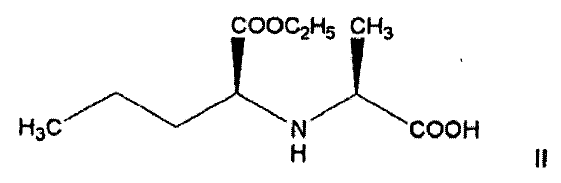 aktiviert wird mit einem Tetramethyluroniumsalz der Formel III 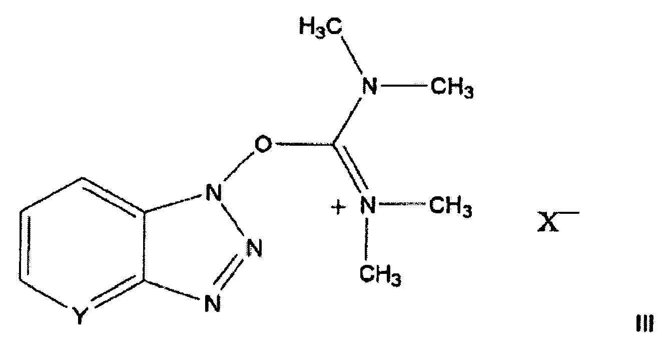 worin Y für ein aromatisches C oder N Atom steht und X⁻ ein Anion ist, wie Tetrafluorborat, Hexafluorphosphat oder Halogen,
und die erhaltene aktivierte Aminosäure der Formel II dann umgesetzt wird mit einer (2S,3aS,7aS)-Octahydroindol-2-carbonsäure oder einem Ester hiervon der Formel IV 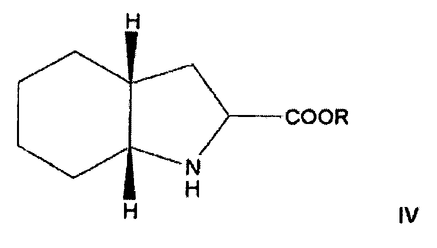 worin R für Wasserstoff, eine Benzylgruppe, eine tertiäre Butylgruppe oder eine Trimethylsilylgruppe steht, und die Schutzgruppe R nach Beendigung der Reaktion durch Hydrierung, Phasentransferhydrierung oder Extraktion entfernt wird.

2. Verfahren nach Anspruch 1, **dadurch gekennzeichnet, dass** das Tetramethyluroniumsalz aus der Gruppe ausgewählt wird, die besteht aus
O-(Benzotriazol-1-yl)-N,N,N',N'-tetramethyluroniumhexafluorphosphat,
O-(Benzotriazol-1-yl)-N,N,N',N'-tetramethyluroniumtetrafluorborat und
O-(7-Azabenzotriazol-1-yl)-N,N,N',N'-tetramethyluroniumhexafluorphosphat.

3. Verfahren nach einem der Ansprüche 1 oder 2, **dadurch gekennzeichnet, dass** die Aktivierung der Carboxylgruppe mit Tetramethyluroniumsalzen durch Verwendung einer tertiären organischen Base promoviert wird.

4. Verfahren nach Anspruch 3, **dadurch gekennzeichnet, dass** die tertiäre organische Base ausgewählt wird aus der Gruppe, die besteht aus N,N-Diisopropylethylamin und Triethylamin.

5. Verfahren nach Anspruch 1, **dadurch gekennzeichnet, dass** die Schutzgruppe R eine Benzylgruppe ist.

6. Verfahren nach Anspruch 5, **dadurch gekennzeichnet, dass** die Benzylschutzgruppe durch Phasentransferhydrierung ohne Verwendung von gasförmigem Wasserstoff entfernt wird.

## Revendications

1. Procédé pour la préparation de périndopril de formule I : 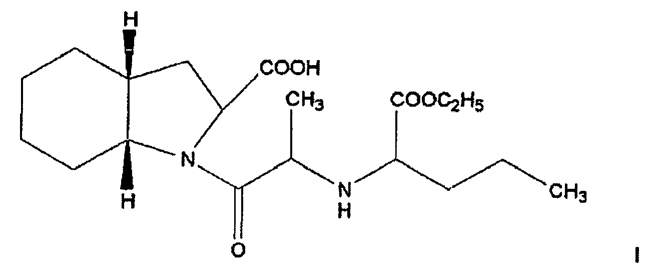 **caractérisé en ce que** le groupe carboxy du N-/1-(S)-éthoxycarbonylbutyl/-(S)-alanine d'acide aminé stéréospécifique de formule II : 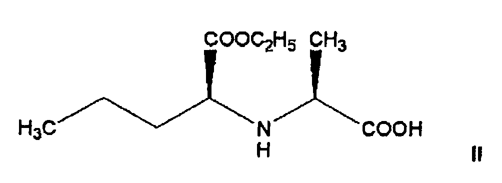 est activé avec un sel de tétraméthyluronium de formule III : 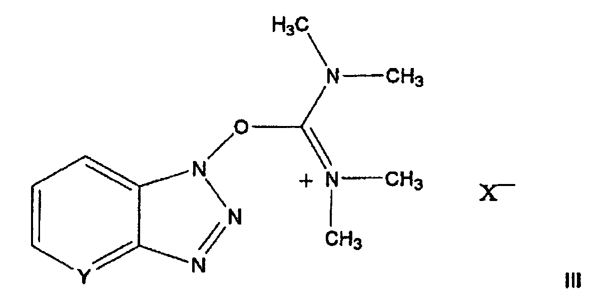 dans laquelle, Y est un atome C ou N aromatique, X⁻ est un anion tel que tétrafluoroborate, hexafluorophosphate ou halogène,
et ensuite, **en ce que** l'acide aminé activé de formule II obtenu est réagi avec l'acide (2S,3aS,7aS)-octahydroindole-2-carboxylique de formule IV : 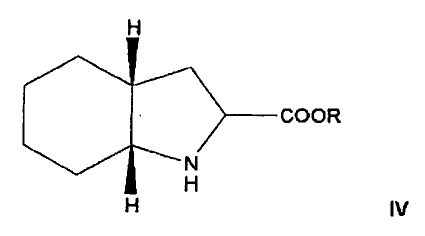 dans laquelle, R est hydrogène, un groupe benzylique, un groupe butylique tertiaire ou un groupe triméthylsilyle,
et, **en ce qu'**une fois la réaction terminée, le groupe protecteur R est éliminé par hydrogénation, hydrogénation par transfert de phases ou extraction.

2. Procédé selon la revendication 1, **caractérisé en ce que** le sel de tétraméthyluronium est sélectionné à partir du groupe composé :
d'hexafluorophosphate de O-(benzotriazol-1-yl)-N,N,N',N'-tétraméthyluronium,
de tétrafluoroborate de O-(benzotriazol-1-yl)-N,N,N',N'-tétraméthyluronium, et
d'hexafluorophosphate de O-(7-aza-benzotriazol-1-yl)-N,N,N',N'-tétraméthyluronium.

3. Procédé selon l'une quelconque des revendications 1 à 2, **caractérisé en ce que** l'activation du groupe carboxylique avec les sels de tétraméthyluronium est favorisée par l'utilisation d'une base organique tertiaire.

4. Procédé selon la revendication 3, **caractérisé en ce que** la base organique tertiaire est sélectionnée à partir du groupe composé de N,N-diisopropyléthylamine et triéthylamine.

5. Procédé selon la revendication 1, **caractérisé en ce que** le groupe protecteur R est un groupe benzylique.

6. Procédé selon la revendication 5, **caractérisé en ce que** le groupe benzylique protecteur est éliminé par hydrogénation par transfert de phase sans utilisation d'hydrogène gazeux.
